Europäisches Patentamt

European Patent Office

Office européen des brevets

(11) Publication number: **0 122 681**
**B1**

(12) **EUROPEAN PATENT SPECIFICATION**

(45) Date of publication of patent specification: 07.01.87

(21) Application number: 84200529.0

(22) Date of filing: 12.04.84

(51) Int. Cl.⁴: **C 12 P 1/00,** C 12 N 11/18, C 12 P 37/06

(54) Enzymatic process.

(30) Priority: 19.04.83 NL 8301373

(43) Date of publication of application:
24.10.84 Bulletin 84/43

(45) Publication of the grant of the patent:
07.01.87 Bulletin 87/02

(84) Designated Contracting States:
AT BE CH DE FR GB IT LI LU NL SE

(56) References cited:
FR-A-2 036 383

EUROPEAN JOURNAL OF BIOCHEMISTRY, vol.
36, no. 1, 1973, pages 89-96; S. GESTRELIUS et
al.: "On the regulation of the activity of
immobilized enzymes. Microenvironmental
effects of enzyme-generated pH changes"

CHEMICAL ABSTRACTS, vol. 87, no. 11, 12th
September 1977, page 227, no. 80462m,
Columbus, Ohio, US; B. ATKINSON et al.:
"Characteristics of unbuffered gel-immobilized
urease particles".

(73) Proprietor: NEDERLANDSE CENTRALE
ORGANISATIE VOOR TOEGEPAST-
NATUURWETENSCHAPPELIJK ONDERZOEK
Juliana van Stolberglaan 148
NL-2595 CL The Hague (NL)

(72) Inventor: Rousseau, Itzhak
Laan van Henegouwen 13
NL-3703 TC Zeist (NL)

(74) Representative: van der Beek, George Frans
et al
Nederlandsch Octrooibureau
Johan de Wittlaan 15 P.O. Box 29720
NL-2502 LS 's-Gravenhage (NL)

**0 122 681**

**Description**

The present invention relates to a process for carrying out an enzymatic process, in which an immobilised biocatalyst containing an enzyme catalysing the formation of a proton generating product, and containing an enzyme catalysing the formation of a proton consuming product is contacted with a medium containing substrates for the enzymes present in the immobilised biocatalyst.

In a study published in Eur.J.Biochem. *36* (1973), pp 89—96 the effects of proton production or consumption by one enzyme upon other enzymes have been investigated. Most of the experiments were carried out by using two enzymes co-entrapped in polyacrylamide particles, and measuring the pH-dependent activity curve of the enzymes in 5 mM buffer. In one of the experiments glucose oxidase was co-entrapped with urease, and the glucose oxidase activity towards glucose was measured at various pH values in 5 mM buffer, using glucose only, and in a further experiment, glucose and urea as a substrate. The pH optimum of glucose oxidase activity appeared to be shifted to the acidic side in the presence of urease activity. On increasing the molarity of the buffer used, from 5 to 50 mM, the pH shift of the activity curve could be overcome.

B. Atkinson, J. Rott and I. Rousseau [Biotechnology and Bioengineering, vol. XIX, pp 1037—1063 (1977)] and B. Atkinson and I. Rousseau [ibid., pp 1065—1086 (1977)] have shown that the internal pH of immobilised urease gel particles in contact with an urea solution generally differs from the external pH* of the substrate solution, unless the external pH* is in the region of 8.83. The internal $pH_i$ is the result of the self-buffering capacity of the reaction products of the urea hydrolysis, viz. ammonia and carbon dioxide.

The process of the present invention is based on the finding that it is possible to use one, auxiliary, enzymatic reaction for generating an internal $pH_i$ favourable for a second, productive enzymatic reaction. The internal $pH_i$ can be maintained exclusively by controlling the reaction conditions of the auxiliary reaction, while external pH control, e.g. by addition of alkali, acid or buffers, the substrate medium, is not used.

Consequently, in the process of the present invention, in which an immobilised biocatalyst containing an enzyme catalysing the formation of a proton generating product, and containing an enzyme catalysing the formation of a proton consuming product is contacted with a medium containing substrates for the enzymes present in the immobilised biocatalyst, pH conditions favourable for one of the enzymatic reactions are maintained exclusively by controlling the concentration of the substrate for the other enzymatic reaction. Further details of the process are set out in the dependent claims 2 to 6.

In the present process, any enzyme capable of catalysing the formation of a proton generating product can be used together with any second enzyme capable of catalysing the formation of a proton consuming product. Purified enzyme preparations may be used, but viable or non-viable enzymatically active microbial cells may be used as well. Industrially important systems are acylases hydrolysing carbon-nitrogen bonds, for example penicillin acylase, cephalosporin acylase and D- or L-aminoacid acylase. These enzymes are being used on a large scale. A further important group of enzymes catalysing the formation of a proton generating product are ester hydrolases and proteases, for example α-chymotrypsin, papain, etc. An important enzyme catalysing the formation of a proton consuming product is urease.

Penicillin acylase is generally obtained from *Escherichia coli*, and is used as a catalyst in the deacylation of penicillins, especially of benzylpenicillin or phenoxypenicillin. This results in the production of 6-aminopenicillanic acid (6-APA), which is an important starting material for semi-synthetic penicillins. The deacylation reaction of benzylpenicillin results in the formation of 6-APA and phenylacetic acid. At the optimum pH of the enzymatic reaction, that is to say at a pH of 8.0, the phenylacetic acid is present mainly in dissociated form. Consequently, in prior art processes, a constant pH can be maintained only by addition of base or by using a large amount of buffer. It is necessary to maintain the pH at the optimum value because, otherwise, the activity and the stability of the enzyme would decrease.

Urease is the preferred enzyme to be used in the present process as an enzyme catalysing the formation of proton consuming products. Urease catalyses the hydrolysis of urea to ammonia and carbon dioxide, products forming a solution having a large buffer capacity. It is highly advantageous to use internal buffering of the immobilised enzyme by the hydrolysis products of urea for controlling the pH of a system in which a proton generating product is formed, for example a system in which penicillin acylase, cephalosporin acylase or an other acylase, an ester hydrolase or protease is in contact with the corresponding substrate. In this manner the internal pH in an immobilised biocatalyst containing urease and penicillin acylase in a matrix of a solid carrier can be controlled. This results in an important improvement of the activity and the stability of the immobilised acylase.

The immobilised biocatalysts to be used in the present process can be prepared by any known immobilisation technique. Generally, such techniques comprise covalent bonding to an insoluble carrier, such as cellulose or amberlite, adsorption to an insoluble carrier, such as bentonite, or entrapment in an insoluble matrix. Entrapment in hollow synthetic resin fibers or in gel particles is widely used. Preferred gel matrices are polyacrylamide gel and glutaric aldehyde cross-linked gelatin gel. Entrapment in cross-linked gelatin particles is most preferred since the method is simple and gives good results.

The invention is further illustrated by a process in which an immobilised biocatalyst containing penicillin acylase and urease is used. It should be clear, however, that the invention is not limited to this process.

2

The optimum pH of most commercial urease preparations is situated between 6.3 and 6.5. The activity of penicillin acylase is influenced by the concentration of the substrate, for example benzylpenicillin. The optimum pH for this enzyme lies at pH = 8. The enzyme is also inhibited by the products, 6-aminopenicillanic acid and phenylacetic acid. 6-Aminopenicillanic acid acts as a non-compatitive inhibitor and phenylacetic acid is a competitive inhibitor. Most probably, the dissociated form of phenylacetic acid is the inhibitor. When the benzylpenicillin concentration is higher than 0.01 M the reaction rate decreases as a result of inhibition by the product.

Experiments were conducted with soluble urease and penicillin acylase to evaluate the pH variations during enzymatic reaction under unbuffered conditions so as to determine whether it would be possible to combine these enzyme systems in such a way that favourable and constant pH conditions could be maintained.

The pH variations were measured with a pH meter coupled to a recorder. The reaction was carried out with urease and with penicillin acylase concentrations of 1 µg/ml. The urea and benzylpenicillin concentrations were 0.01 M. The substrates were dissolved in distilled water. The pH of the solution was adjusted either with acid or alkali to an initial pH of 7.0. When only urease was added to the above mentioned mixed substrate solution, the reaction product, ammonia, caused a decrease of the proton concentration resulting in an increase of the pH to 8.77. When only penicillin acylase was added to the mixed substrate solution at pH 7, the pH decreased to a value of 4.91

The products of the enzymatic hydrolysis of urea, that is to say ammonia and carbon dioxide, produced a solution having a self-buffering capacity at pH 9.0, and the products of the deacylation of benzylpenicillin formed a solution having a buffering capacity at pH 4.5.

In the simultaneous reaction of the two enzymes using an urease concentration of 10 µg/ml and a penicillin acylase concentration of 100 µg/ml, the pH of the solution reached a value of 7.8 in 20 minutes. Increasing the penicillin acylase concentration to 200 µg/ml suppressed the pH variation, the pH remaining at a value of 7.0 for more than 20 minutes. These experiments demonstrate that combination of the two enzymes provides a system in which pH variations may be suppressed.

When carrying out the enzymatic reaction according to the invention the molar ratio of the substrates is selected in such a way that the pH is maintained at a value in the region of optimum productivity. In the case of penicillin acylase and urease, it has appeared that equimolar ratios of penicillin and urea substrates will lead to a practically constant pH in the optimum region for benzylpenicillin deacylation.

The substrate concentration in the substrate medium is not critical. Naturally, the highest possible concentration will be used. Good results have been obtained with about 0.2 molar benzylpenicillin concentrations.

The enzymatic reaction may be carried out batchwise or continuously. Continuous operation can be effected in fluidised bed reactors or fixed bed reactors. Fixed bed reactors are the most exploited type of reactors in immobilised enzyme processes. There are several reasons for the preference of a fixed bed reactor. These reasons are: a high efficiency, a simple design and an easy mode of operation. A serious disadvantage of prior art enzymatic processes in fixed bed reactors is non-effective pH control. The deacylation of penicillin cannot be carried out in a single-stage fixed bed reactor due to a marked decrease of activity and enzyme stability caused by the axial pH drop produced by the phenylacetic acid or other acid formed during the deacylation. As stated in Biotechnology and Bioengineering vol. XXV, pp 1873—1895 (1983) multi-stage fixed bed reactors with adjustment of pH between reactors should be used. This disadvantage of prior art processes is eliminated by the present process in which internal pH control in the immobilised enzyme particles is used. The present process may be carried out successfully in a single-stage fixed bed reactor.

Continuous operation of the co-immobilised enzyme system can be maintained for long periods of time. The penicillin acylase activity declined only slightly in a period of 800 hours. In this time, a 10% loss in the enzyme activity occurs. Thus, both enzymes can react simultaneously for long periods of time maintaining an internal pH control. The reaction products of urease provide microenvironmental pH conditions which are near the optimum for the activity of penicillin acylase.

In addition to the intra-particle pH control the co-immobilised system provides a pH control along the axial direction of the fixed bed column. Internal pH control allows deacylation of high concentrations of benzylpenicillin at high conversion rates. These improvements in the enzymatic deacylation of penicillin lead to substantial reduction in the costs of the production and the recovery of 6-APA.

The co-immobilised enzyme systems can be operated at a near optimum pH if equimolar concentrations of both substrates are used. A reduction in the urea concentration leads to a lower pH and, consequently, to a decrease in the deacylation rate.

Introduction of the urea hydrolysis products ammonium carbonate and ammonium bicarbonate at a relatively high concentration, i.e. 0.4 M, could not maintain the intra-particle pH at the required value. This evidently demonstrates the effectiveness of internal generation and outward diffusion of pH controlling ionic species.

Another advantage of the present process is that the pH in the feed solution may be maintained at values at which negligeable pH dependent side reactions occur, without affecting the conversion.

The following examples illustrate the process of the invention. These examples are exemplary only to show some of several possible ways of carrying out the present process.

## Example 1

Preparation of the immobilised biocatalyst

A gelatin solution was prepared by dissolving 20% (w/v) of gelatin in 0.1 M phosphate buffer, pH = 6.0. The solution was heated to 60°C to promote dissolution.

Penicillin acylase (100 mg/ml) and urease (30 mg/ml) were dissolved in phosphate buffer, pH = 6.0. The solution was centrifuged to remove cell debris from the penicillin acylase preparation.

The gelatin and enzyme solutions were mixed in equal volumes at 40°C. The mixture was added dropwise to cold butyl acetate (ice bath) to form spherical gel particles having a diameter of about 3 mm. The gel particles were removed from the butyl acetate and cross-linked in a 2% (w/v) solution of glutaraldehyde for 30 minutes at room temperature (about 18°C) or at 4°C.

The cross-linked enzyme gel particles were washed several times with phosphate buffer, pH = 6 and stored in the same buffer at 4°C. The particles contained 10% (w/v) gelatin, 50 mg/g penicillin acylase and 15 mg/g urease.

Use of the immobilised biocatalyst

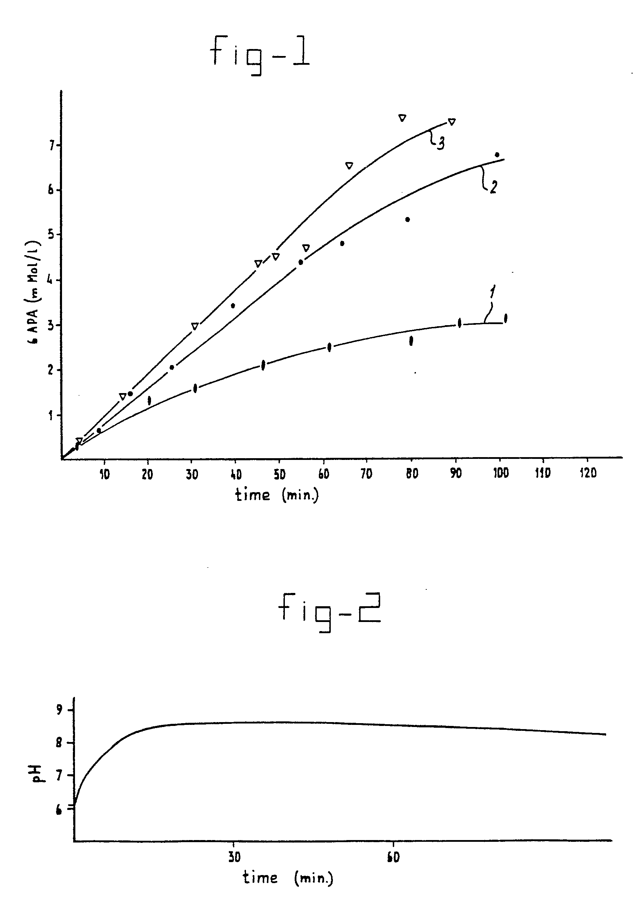

A batch of 1.0 g of the particles prepared in the above mentioned way was introduced into a reactor of 50 ml. A substrate solution (25 ml) was added and the temperature was maintained at 37°C. The concentration of the 6-aminopenicillanic acid (6-APA) was determined as a function of time. The values found are given in Fig. 1.

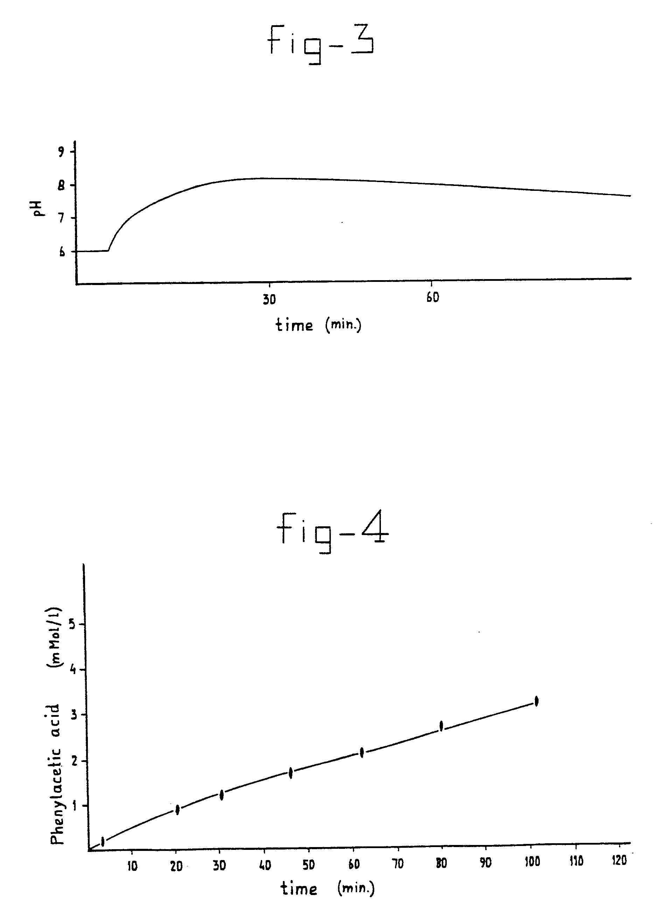

In a first experiment the substrate solution was 0.02 M benzyl penicillin in distilled water, and the pH was maintained at 8.0 by addition of alkali. The result is given in curve 1 of Fig. 1. The curve shows that the production of 6-APA decreases rapidly with time. The phenylacetic acid production in this experiment as a function of time is shown in Fig. 4. The concentration of the phenylacetic acid was calculated from the amounts of alkali added.

In a second experiment the substrate solution was 25 ml of 0.02 M benzylpenicillin and 0.01 M urea in distilled water. As shown by curve 2 of Fig. 1 the activity of the penicillin acylase is increased 2.5 times. The pH variation during the reaction of this experiment appears from Fig. 2. Initially, the pH increased to pH 8.5 to 9.0 in 15 minutes, a value at which the products of urea hydrolysis exhibit self-buffering capacity. It may be assumed that the increased pH is the result of the fact that urea diffuses more easily than benzylpenicillin. Then the pH gradually decreased to 8.15 in 170 minutes.

In a further experiment the substrate solution was 0.2 M benzylpenicillin and 0.005 M urea in distilled water. This resulted in a threefold increase of the activity of the penicillin acylase, as is shown by curve 3 of Fig. 1. The corresponding pH-graph, Fig. 3, shows that the pH gradually increased to 8.0 in 45 minutes and then slowly decreased to 7.5.

The immobilised enzyme particles did not show loss of activity after 200 hours of continuous use. The results given in Fig. 1 show clearly that the internal pH control as a result of buffering with the reaction products of the enzymatic urea hydrolysis lead to an increase of the immobilised penicillin acylase activity.

## Example 2

Continuously stirred reactor (CSTR)

This example describes the deacylation of benzylpenicillin (BP) in a 50 ml thermostated (37°C) continuously stirred reactor (CSTR). The pH of the reaction was controlled internally by feeding urea together with the benzylpenicillin. Operation parameters used in the CSTR study are listed in Table 1.

The cross-linked gelatin gel particles were prepared as described in Example 1. The enzyme concentration in the gel particles is shown in Table 1.

To avoid contamination, the reactor system was autoclaved and the substrate solution was filtered via 0.22 μm filter. Gel particles were transferred aseptically from the glutaraldehyde solution into the reactor.

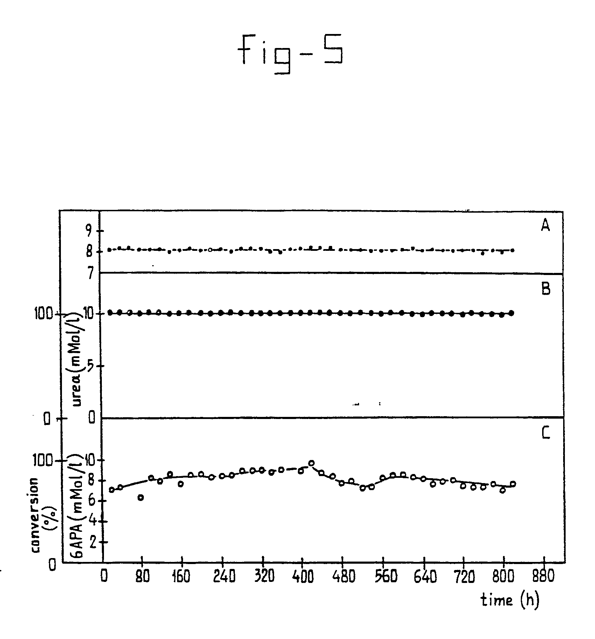

Fig. 5 illustrates aseptic operation for more than 740 hours of the co-immobilised system exposed to 10 mM benzylpenicillin and 10 mM urea. A shows the bulk solution pH*; B shows urea concentration and conversion; and C shows 6-APA concentration and conversion. Other experimental parameters are given in Table 1. The concentration of penicillin acylase was 1 mg (enzyme)/g (gel). A constant conversion of about 85% was observed in the first 550 hours of operation. Thereafter a gradual decrease in conversion occurred. The pH was maintained at a constant value of about 8.0 during the 800 hours of operation. Fig. 5 clearly demonstrates that the two immobilised enzymes can react and operate simultaneously for extensive periods of time with internal pH-control.

## Example 3

Fixed bed reactor

In this example, the deacylation of benzylpenicillin was effected in a fixed bed reactor. This was a thermostated (37°C) chromatography column having a height of 14.5 cm and an internal diameter of 2.4 cm which was initially filled with a 5 cm layer of glass beads. On top of the beads the gel particles were packed. The feed solution was pumped at constant rate using a peristaltic pump. Effluent stream was collected in a sample collector and samples were analysed for pH, 6-APA and $NH_3$-formation. The reactor had a liquid volume of 11.54 ml and a gel bed of 17,0 g. In these experiments the co-immobilised system was exposed to 100 mM benzylpenicillin. The various experimental parameters involved in these experiments are summarised in Tables 1 and 2.

The effect of the urea concentration variation in the feed on the reactor bulk pH* and consequently on the substrate conversion is shown in Table 2 at 6.5 h. A decrease in the urea concentration from 100 mM to 50 mM, at a constant benzylpenicillin concentration of 100 mM, resulted in a pH* drop from 8.5 to 6.5 and in a lower degree of substrate conversion of about 38.7%.

To test the performance of a fixed bed column with high substrate concentration, the concentrations of both benzylpenicillin and urea were increased to 200 mM. Table 2 at 30 h demonstrates that a lower conversion (about 40%) was obtained with these high concentrations as compared with 100 mM benzylpenicillin, using a similar flow-rate. This low value of conversion is probably due to the low enzyme load in the column, hence an insufficient activity of penicillin acylase. The activity of urease, however, was sufficient to maintain a constant pH of 8.5.

The influence of the feed flow-rates, expressed as space velocity, on the substrate conversion is illustrated by the values at 37, 44, 52, and 81 h in Table 2. Increasing the fluid space velocity in the column results, as expected, in a lower degree of conversion. At a space velocity of 3.6 h$^{-1}$ only 52,4% conversion was obtained with 100 mM benzylpenicillin. Low substrate conversions were usually coupled to an increase in the bulk solution pH*. This indicates that urease, which is in excess in the particle and has a higher specific activity, can tolerate higher space velocities. Thus, in order to get a better performance at high space velocities, high concentrations of penicillin acylase should be used.

Effects of external buffering on the performance of the fixed bed column are shown by the values at 22 and 88 h in Table 2. Tris buffers of 0.1 M and 0.3 M, respectively, were used. The use of 0.1 M Tris buffer could not compensate for the acid generated by the deacylation reaction; consequently the pH decreased to below 6.0 and a low conversion of 35.0% was obtained. Increasing the buffer strength to 0.3 M improved the buffering capacity of the solution and a conversion of about 50% could be obtained. Still, a concentration of 0.3 M Tris buffer could not compensate for the phenylacetic acid formed during the reaction, as reflected by the solution pH*, which decreased to about 7.2.

In a final experiment with this column after a total of 120 working hours, the bed was exposed to only 100 mM benzylpenicillin, omitting buffer or urea. A pH-drop to about 5.0 was observed after 26 hours and the conversions of benzylpenicillin were below 10%.

Under conditions of no pH-control, the products of benzylpenicillin deacylation form a buffer at pH* = 4.5.

## Example 4

In the previous set of experiments it was observed that a penicillin acylase loading of 34.0 mg (enzyme)/column was not sufficient to maintain a high conversion of 200 mM benzylpenicillin. In order to enhance the conversion of the reactor, its bed volume was increased to 57,0 g and the load of penicillin acylase to 5 mg (enzyme)/g (gel). More details of the experimental parameters of this fixed bed are given in Table 1.

The influence of the feed flow-rate on the conversion of 200 mM benzylpenicillin in the feed is shown in Table 3. At a space velocity of 6.0 h$^{-1}$ a conversion of more than 80% of benzylpenicillin was obtained. An increase in the flow-rates was coupled with a decrease in the degree of conversion. Thus, a reduction in the production rate of phenylacetic acid, not coupled to a similar reduction of urea hydrolysis, resulted in an increase in the bulk solution pH*, as summarised in Table 3.

Operation of the column at different (lower) urea concentrations at a constant flow-rate led to a sharp decrease in the degree of benzylpenicillin conversion. This decrease in the conversion is a result of a reduction in the buffer capacity of the system. Thus, lowering of the $pH_i$ within the gel particles has a significant effect on the rate of reaction of penicillin acylase. The results of replacing urea by the hydrolysis products $NH_4HCO_3$ in the feed are given in Table 3. Addition of either $(NH_4)_2CO_3$ or $NH_4HCO_3$ could not provide a sufficiently buffered system and, consequently, lower pH* values and conversions were obtained by the fixed bed reactor. In the case of $NH_4HCO_3$ the conversions were 35.0% and 39.6% using 0.2 M and 0.4 M, respectively (see Table 3). When 0.2 M or 0.4 M $(NH_4)_2CO_3$ were used, the conversions were 36.6% and 40.0%, respectively. $(NH_4)_2CO_3$ has a better buffering capacity and can maintain the pH at values betwen 7.5 and 8.3, using the above concentrations.

The operation of the column for more than 75 hours led to about 25% loss in activity under these conditions of operation.

## TABLE 1
### Experimental Parameters of Co-Immobilised Enzyme System

| Fig. | conc. of PA mg/g (gel) | conc. of urease mg/g (gel) | amount of gel in reactor (g) | vol. of liquid in reactor (ml) | reactor type | feed flow-rate ml/h |
|---|---|---|---|---|---|---|
| 5 | 1.0 | 25.0 | 17.0 | 48.0 | CSTR | 30 |
|  | 2.0 | 23.0 | 17.0 | 11.54 | fixed | variable see Table 2 |
|  | 5.0 | 20.0 | 57.0 | 8.0 | fixed | variable see Table 3 |

## TABLE 2
### Summary of Operational Parameters of a Fixed Bed Reactor with a Liquid Volume of 11.54 ml and a Gel Bed of 17.0 g

| actual operation time (h) | BP (mM) | urea (mM) | feed flow-rate (ml/h) | space velocity $(h^{-1})$ | pH* | Conversion (%) | buffer, if any |
|---|---|---|---|---|---|---|---|
| 6.5 | 100 | 50 | 21.0 | 1.82 | 6.50 | 38.7 | — |
| 15 | 100 | 100 | 21.0 | 1.82 | 8.15 | 84.0 | — |
| 22 | 100 | — | 18.0 | 1.56 | 5.45 | 35.0 | Tris, 0.1 M |
| 30 | 200 | 200 | 19.8 | 1.72 | 8.50 | 39.9 | — |
| 37 | 100 | 100 | 19.8 | 1.72 | 8.15 | 74.4 | — |
| 44 | 100 | 100 | 42.0 | 3.64 | 8.65 | 52.4 | — |
| 52 | 100 | 100 | 57.0 | 4.94 | 8.80 | 41.4 | — |
|  | 100 | 100 | 7.8 | 0.68 | 8.30 | 75.3 | — |
|  | 100 | 100 | 51.0 | 4.42 | 8.95 | 41.4 | — |
| 81 | 100 | 100 | 120.0 | 10.40 | 9.15 | 16.8 | — |
| 88 | 100 | — | 19.8 | 1.72 | 7.25 | 46.9 | Tris, 0.3 M |
| 120 | 100 | — | 19.8 |  | 5.66—4.82 | 30.0—10.0 | — |

TABLE 3
Summary of Operational Parameters of a Fixed Bed Reactor with a Liquid Volume of 8.0 ml and a Gel Bed of 57.0 g

| elapsed operation time (h) | BP (mM) | urea (mM) | feed flow-rate (ml/h) | space velocity ($h^{-1}$) | pH* | conversion % | buffer, if any |
|---|---|---|---|---|---|---|---|
| 32.5 | 200 | 200 | 48.0 | 6.0 | 8.20 | 79.9 | — |
| 34.5 | 200 | 200 | 111.0 | 13.88 | 8.95 | 56.6 | — |
| 36.0 | 200 | 200 | 232.8 | 29.10 | 9.20 | 29.1 | — |
| 53.0 | 200 | 200 | 46.8 | 5.85 | 8.65 | 68.3 | — |
| 57.0 | 200 | — | 48.0 | 6.0 | 6.75 | 31.8 | $NH_4HCO_3$, 0.2 M |
| 60.0 | 200 | — | 48.0 | 6.0 | 7.35 | 39.6 | $NH_4HCO_3$, 0.4 M |
| 64.0 | 200 | 200 | 48.0 | 6.0 | 8.55 | 70.6 | — |
| 67.5 | 200 | 100 | 49.8 | 6.23 | 7.25 | 52.5 | — |
| 71.5 | 200 | 50 | 48.6 | 6.08 | 6.30 | 29.1 | — |
| 75.5 | 200 | 200 | 48.6 | 6.08 | 8.50 | 61.1 | — |
| 82.5 | 200 | — | 48.6 | 6.08 | 7.45 | 36.6 | $(NH_4)_2CO_3$, 0.2 M |
| 89.5 | 200 | — | 49.5 | 6.19 | 7.90 | 35.5 | $(NH_4)_2$, 0.4 M |

## Claims

1. A process for carrying out an enzymatic process, in which an immobilised biocatalyst containing an enzyme catalysing the formation of a proton generating product, and containing an enzyme catalysing the formation of a proton consuming product is contacted with a medium containing substrates for the enzymes present in the immobilised biocatalyst, characterized by maintaining pH conditions favourable for one of the enzymatic reactions exclusively by controlling the concentration of the substrate for the other enzymatic reaction.

2. The process of claim 1, in which the substrate for one of the enzymatic reactions is a penicillin or a cephalosporin, and the substrate for the other enzymatic reaction is urea.

3. The process of claim 2, in which the substrates are benzylpenicillin and urea.

4. The process of claim 3, in which an immobilised biocatalyst containing penicillin acylase and urease is contacted with a medium containing benzylpenicillin and urea, and that the urease concentration in the biocatalyst and the urea concentration in the substrate medium are such that the pH in the biocatalyst is near the optimum for the benzylpenicillin deacylation.

5. The process of claim 4, in which the benzylpenicillin and urea concentrations in the substrate medium are equimolar.

6. The process of claim 1, in which the immobilised biocatalyst contains the enzyme entrapped in a glutaraldehyde cross-linked gelatin gel.

## Patentansprüche

1. Verfahren zur Durchführung eines enzymatischen Prozesses, bei welchem ein immobilisierter Biokatalysator, der ein Enzym, das die Bildung eines protonbildenden Produktes katalysiert, und ein Enzym, das die Bildung eines protonverbrauchenden Produktes katalysiert, enthält, mit einem Medium kontaktiert wird, das Substrate für die im immobilisierten Biokatalysator anwesenden Enzyme enthält, dadurch gekennzeichnet, daß man pH-Bedingungen, die für eine der enzymatischen Reaktionen günstig sind, ausschließlich aufrechterhält, indem man die Konzentration des Substrates für die andere enzymatische Reaktion regelt.

2. Verfahren nach Anspruch 1, bei welchem das Substrat für eine der enzymatischen Reaktionen ein Penicillin oder ein Cephalosporin und das Substrat für die andere enzymatische Reaktion Harnstoff ist.

3. Verfahren nach Anspruch 2, bei welchem die Substrate Benzylpenicillin und Harnstoff sind.

4. Verfahren nach Anspruch 3, bei welchem ein immobilisierter Biokatalysator, der Penicillinacylase und Urease enthält, mit einem Medium kontaktiert wird, das Benzylpenicillin und Harnstoff enthält, und daß die Ureasekonzentration im Biokatalysator und die Harnstoffkonzentration im Substratmedium so bemessen sind, daß sich der pH-Wert im Biokatalysator nahe des Optimums für die Benzylpenicillindeacylierung befindet.

5. Verfahren nach Anspruch 4, bei welchem die Benzylpenicillin- und Harnstoffkonzentrationen im Substratmedium äquimolar sind.

6. Verfahren nach Anspruch 1, bei welchem der immobilisierte Biokatalysator die Enzyme, eingeschlossen in einem mit Glutaraldehyd vernetzten Gelatinegel, enthält.

**Revendications**

1. Procédé pour la réalisation d'un processus enzymatique, dans lequel un biocatalyseur immobilisé contenant une enzyme catalysant la formation d'un produit générant des protons et contenant une enzyme catalysant la formation d'un produit consommant des protons est mis en contact avec un milieu contenant des substrats pour les enzymes présentes dans le biocatalyseur immobilisé, caractérisé en ce que des conditions de pH favorables pour l'une des réactions enzymatiques sont maintenues exclusivement en contrôlant la concentration du substrat pour l'autre réaction enzymatique.

2. Procédé de la revendication 1, dans lequel le substrat pour l'une des réactions enzymatiques est une pénicilline ou une céphalosporine et le substrat pour l'autre réaction enzymatique est l'urée.

3. Procédé de la revendication 2, dans lequel les substrats sont la benzylpénicilline et l'urée.

4. Procédé de la revendication 3, caractérisé en ce qu'un biocatalyseur immobilisé contenant la pénicilline acylase et l'uréase est mis en contact avec un milieu contenant la benzylpénicilline et l'urée, et en ce que la concentration de l'uréase dans le biocatalyseur et la concentration de l'urée dans le milieu de substrats sont telles que le pH dans le biocatalyseur est voisin de l'optimum pour la désacylation de la benzylpénicilline.

5. Procédé de la revendication 4, dans lequel les concentrations de benzylpénicilline et d'urée dans le milieu de substrats sont équimolaires.

6. Procédé de la revendication 1, dans lequel le biocatalyseur immobilisé contient les enzymes incluses dans un gel de gélatine réticulée au glutaraldéhyde.

# Fig-1

# Fig-2

# Fig-3

# fig-4

Fig-5